# EUROPEAN PATENT APPLICATION

(11) **EP 4 703 458 A1**
(43) Date of publication of application: **04.03.2026**
(21) Application number: 24197604.2
(22) Date of filing: 30.08.2024
(51) Int. Cl.: C10L 1/10, C10L 1/18, C10L 1/183, C10L 1/189, C10L 1/19, C10L 1/26, C07C 69/00, C08K 5/00

(54) **LEVULINATE ESTER COMPOSITION COMPRISING A PHENOLIC ANTIOXIDANT**

(71) Applicant: GFBiochemicals IP Assets B.V., 6167 RD Geleen (NL)
(72) Inventor: Grapeloup, Cécile, 13008 Marseille (FR); Chantreux, Mathilde, 13004 Marseille (FR); Parton, Rudy, 3020 Winksele (BE); Carotenuto, Giuseppina, 13100 Aix en Provence (FR)
(74) Representative: Fiorucci, Hélène

(57) **Abstract**

This invention relates to a levulinate ester composition comprising a levulinate ester; and at least one phenolic antioxidant. The phenolic antioxidant is preferably at least one of tocopherol, a derivative of tocopherol, tocotrienol, hydroquinone, an alkyl phenol, butylhydroxytoluene (BHT), and mixture thereof. The present invention also relates to a method for preparing such composition, as well as to the use of at least one phenolic antioxidant to reduce or avoid the color development in levulinate ester compositions.

## Description

### FIELD OF THE INVENTION

This invention relates to a levulinate ester composition comprising a levulinate ester; and at least one phenolic antioxidant. The phenolic antioxidant is preferably at least one of tocopherol, a derivative of tocopherol, tocotrienol, hydroquinone, butylhydroxytoluene (BHT), and mixtures or combinations thereof. The present invention also relates to the use of at least one phenolic antioxidant to reduce or avoid the color development in levulinate ester compositions.

### BACKGROUND

Levulinates are important chemicals (Ejaz Ahmad, et al., "Catalytic and mechanistic insights into production of ethyl levulinate from biorenewable feedstocks", Green Chem., 2016, 18, 4804-4823,) utilized as solvents across various markets, including paints and coatings, agro-industry, cleaning and cosmetics. They are also used as intermediates for producing other valuable products such as succinic acid, methyltetrahydrofuran and gamma-valerolactone. The latter can be used to make chemicals such as adipic acid and caprolactam, which in turn serve as monomers for polyesters and polyamides. Additionally, levulinic acid esters themself can be used as fuel or fuel additives for both gasoline based and diesel engines. For several of these applications, a colorless and color-stable levulinic acid ester is a requirement or preferred.

Levulinic acid-based esters can be produced through several methods. One approach involves esterification of levulinic acid (e.g., US 2,029,412; Sindi Baco, et al., "Solvent effect investigation on the acid-catalyzed esterification of levulinic acid by ethanol aided by a Linear Solvation Energy Relationship", Chemical Engineering Science, 2022, pp.117928). Another approach is the direct conversion of hexose sugars in the presence of alcohols to produce levulinate esters (e.g., Yao-Bing Huang, et al., "Facile and high-yield synthesis of methyl levulinate from cellulose", Green Chem., 2018, 20, 1323-1334; Anna Maria Raspolli Galletti, et al., "Direct Alcoholysis of Carbohydrate Precursors and Real Cellulosic Biomasses to Alkyl Levulinates a critical review", Catalysts, 2020, 10(10): 1221). Additionally, levulinic acid-based esters can be synthesized by converting furfuryl alcohol in the presence of alcohols (US 2023/0322655 A1, Rudy Parton and Arie de Rijke, "Process for the conversion of furfuryl alcohol into a levulinate ester"), as byproduct in the synthesis of alkoxymethyl furfuraldehyde (R.J.H. Grisel, et al., "Acid catalysed alcoholysis of wheat straw Towards second generation furan-derivatives", Catalysis Today, 2014, 223, 3- 10). Another approach is the conversion of hydroxymethylfurfural (HMF) or its derivatives with alcohols. Aside from these renewable routes, a petrochemical method exists through the saponification of acetyl succinates (e.g., US 5,189,215). Furthermore, levulinic acid esters can be transesterified with alcohols to produce various levulinates.

Although levulinic esters are virtually colorless immediately after synthesis and purification, they tend to develop a yellow color over time, even following state-of-the-art fractional distillation. Additionally, several methods that produce colorless products immediately after synthesis do not ensure that they will remain colorless over time (e.g., US 2,349,514).

Most technologies for removing the color have been developed for levulinic acid. For example, in CN113929571A, colored species in levulinic acid are removed via adsorption on onto activated carbon or activated clay in an adsorption column, or by oxidization with hypochlorite. Prior to adsorption, the pH of the levulinic acid solution is adjusted with inorganic strong bases. Following treatment, the solution is filtered on a nanofiltration membrane and further purified via distillation. This technology entails considerable additional investment costs. Various other methods have been developed for reducing the color in levulinic acid. They for example involve double extraction combined with a membrane filtration (EP 3823952B1), nanofiltration (WO 2014/037560 A1), crystallization (US 2,305,738, US 2016/0031788 A1, US 10,550,067 B2), treatment withe sunlight (CN112794800A). While these methods effectively reduce the color of the levulinic acid immediately after treatment, they do not guarantee that the color will not re-develop, potentially occurring relatively quickly over time.

Very few technologies address color stability over time. One exception is described in US 2,349,514 where both unwanted odor and undesirable color in levulinic acid are addressed through a treatment with hypochlorite. While the treatment effectively removes the odor, the color tends to reappear rapidly. The yellow color can be more permanently removed via a treatment with hydrogen peroxide. However, the procedure is time consuming and not recommended.

For levulinic acid esters, also known as levulinates, there is very little prior art available. In 1967, Quaker Oats (US 3,358,014) developed a process to make color stable levulinates from furfuryl alcohol-derived levulinates. The method involves treating the color unstable ester at an elevated temperature (above 75°C, preferably 110°C) for an hour in the presence of sulfuric acid (above 0.2 wt.%, preferably 1 wt.%), followed by neutralization and distillation. Not only this technology involves a corrosive process step, but it also entails considerable additional investment costs. Alternatively, adsorption on a column with activated carbon (US 2,029,412) has been described. However, the technology does not address the issue of color stability over time.

Levulinic acid esters are generally considered to be stable and not particularly prone to radical reactions. However, impurities may be present in levulinate esters depending on the manufacturing processes and raw materials used. These impurities may possess different properties that can impact the overall stability of the product, particularly affecting its color. The color of the levulinate ester products may also be impacted by the temperature at which the products are stored. The specific nature of these impurities and what triggers color changes in levulinate esters are not well understood, creating challenges in managing their impact on the levulinate ester product's performance.

The object of the present invention is to reduce or avoid the color development in levulinate ester compositions while minimizing equipment investment and requiring as few additional process steps as possible. The solution provided must not only be easy and cost-effective to implement but must also perform satisfactorily across various temperature conditions, as it should produce a positive impact on the product after manufacturing but also generally contribute to an overall improvement in the levulinate ester products shelf life.

### BRIEF SUMMARY

The present invention relates to a levulinate ester composition comprising a levulinate ester; and at least one phenolic antioxidant.

In some embodiments, the amount of levulinate ester in the composition is at least 95 wt.%, based on the total weight of the composition.

In some embodiments, the amount of phenolic antioxidant is from 10 ppm to 5,000 ppm, based on the total weight of the composition.

In some embodiments, the phenolic antioxidant is at least one of a tocopherol, a derivative of tocopherol, tocotrienol, hydroquinone, an alkyl phenol, butylated hydroxy toluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and any derivative of such compounds, and mixtures or combinations thereof.

The present invention also relates to the use of at least one phenolic antioxidant to reduce or avoid the color development in levulinate ester compositions.

### DETAILED DESCRIPTION

In the present application:
- the expression "comprised between ... and ... " or "from ... to... " should be understood as including the limits;
- any description, even though described in relation to a specific embodiment, is applicable to and interchangeable with other embodiments of the present invention;
- where an element or component is said to be included in and/or selected from a list of recited elements or components, it should be understood that in related embodiments explicitly contemplated here, the element or component can also be any one of the individual recited elements or components, or can also be selected from a group consisting of any two or more of the explicitly listed elements or components; any element or component recited in a list of elements or components may be omitted from such list; and
- any recitation herein of numerical ranges by endpoints includes all numbers subsumed within the recited ranges as well as the endpoints of the range and equivalents.

### Definitions

The term "antioxidant" refers to any compound that prevents or slows the oxidation of other molecules through various mechanisms, including but not limited to radical scavenging. This term highlights the overall function of preventing oxidative damage, which can involve chelating metal ions, decomposing peroxides, or enhancing the activity of other antioxidants.

The term "radical scavenger" denotes a compound that neutralizes free radicals by donating an electron or a hydrogen atom, emphasizing the action of capturing and neutralizing these radicals. Therefore, in the context of the present invention, the term "radical scavenger" is encompassed by the term "antioxidant".

The term "levulinate ester" should be interpreted as having its broadest meaning in the context of the present invention. As such, the levulinate ester may be a levulinate ester with one ester group (also called levulinate monoester), a dilevulinate ester (i.e., a compound formed when levulinate ester is esterified with a glycol), or a multilevulinate ester (i.e., a compound formed when levulinate ester is esterified with a polyol, for example with sorbitol). Preferably, the levulinate ester is a levulinate monoester or a mixture of levulinate monoesters.

The term "phenolic" or "phenolic group" refers to compounds or substances that contain one or several phenol groups. A phenol group consists of a hydroxyl group (-OH) attached to a benzene ring., wherein the hydroxyl group (-OH) is directly bonded to the aromatic benzene ring. Therefore, the phenolic compound may comprise one of several hydroxyl groups, as well as one or several benzene rings. For example, the phenolic compound may be a polyphenolic compound.

The term "phenolic antioxidant" refers herein to an antioxidant, the structure of which comprises a phenolic group. This means that the antioxidant generally comprises other groups or moieties. The nature and number of additional groups or moieties is not limited.

The term "catechins" or "catechin antioxidant" refers to polyphenolic compounds or substances belonging to the flavonoid family. One example of catechin is epigallocatechin gallate (EGCG), which is abundant in green tea.

The term "alkyl phenol" or "alkyl phenol antioxidant" refers to compounds or substances that contain one or several phenol groups, as well as one or more alkyl groups attached to the aromatic ring.

The term "derivative" or "derivative of phenolic antioxidant" refers to a chemically modified version of the phenolic antioxidant of the present invention, retaining the same technical effect. Derivatives may include, for example, salts, esters, ethers, or other chemical modifications that maintain the antioxidant properties of the original compound.

The term "about" means the recited number ± 10%. For example, "about 100" means 90-110, inclusive.

The term "aliphatic" means non-aryl and encompasses non-aromatic rings (e.g., a cycloaliphatic ring). It may be linear or branched, saturated or unsaturated, cyclic or acyclic. An aliphatic ligand may be substituted by one or more groups, for example selected from alkyl, hydroxyl, halogen (Br, Cl, I), isocyanate, carbonyl (=O), amine, carboxylic acid, -C(=O)-OR', -C(=O)-O-C(=O)-R', each R' being independently a C1-C6 alkyl. It may comprise one or more bonds selected from ether, ester, amide, urethane, carbo amide, carbonate, organosiloxane, and mixtures thereof. Examples of aliphatic groups are C1-C6, C1-C10 and C1-C20 aliphatic chains.

The term "aryl" means comprising an optionally substituted polyunsaturated aromatic group. The aryl may contain a single ring (i.e. phenyl) or more than one ring wherein at least one ring is aromatic. When the aryl comprises more than one ring, the rings may be fused or linked via a direct bond (for example biphenyl). The aromatic ring may optionally comprise one to two additional fused rings (i.e. cycloalkyl, heterocycloalkyl or heteroaryl). Examples include phenyl, naphtyl, biphenyl, phenanthrenyl and naphthacenyl.

The term "heteroaryl" means comprising an optionally substituted polyunsaturated aromatic group wherein one or more of the ring atoms is a heteroatom such as O, N and S.

The term "aralkyl" means an aryl substituted by an alkyl group. An example of an aralkyl group is tolyl.

The term "alkaryl" means an alkyl substituted by an aryl group. An example of an alkaryl group is benzyl (-CH₂-Phenyl).

The term "heteroaralkyl" means an aralkyl group in which the aryl contains at least one heteroatom, such as O, N or S.

The term "alkheteroaryl" means an heteroaryl group substituted by an alkyl.

### Levulinate ester composition

The present invention relates to a levulinate ester composition. This levulinate ester composition comprises a levulinate ester and at least one phenolic antioxidant. The composition of the present invention is a liquid.

The inventors have found that adding a phenolic antioxidant to levulinate ester products has an effect in minimizing or preventing yellowing of the products. The solution not only produces an effect on the products immediately after manufacturing, but also over time as it has been shown to attenuate the increase in yellow color over time (e.g., after 35 days). The addition of phenolic antioxidant(s) in levulinate ester compositions, which are stored at high temperatures (i.e., temperatures higher than room temperature, e.g., temperatures above 30°C), has also been shown to be effective in attenuating the increase in color compared to compositions not comprising the phenolic antioxidant.

The addition of a phenolic antioxidant to levulinate ester products meets the objectives of the present invention, as the solution is easy to implement, cost-effective as it does not require significant equipment investments. The invention is inventive and addresses the technical problem effectively.

In some embodiments, the levulinate ester component is the primary or major component in the composition of the present invention. In other words, it forms the largest portion compared to other components in the composition described in the present invention. For example, the amount of levulinate ester in the composition may be at least 95 wt.%, based on the total weight of the composition.

In some other embodiments, the amount of levulinate ester in the composition may be at least 98 wt.%, 98.5 wt.%, at least 99 wt.%, at least 99.5 wt.%, based on the total weight of the composition.

The levulinate ester composition comprises less than 100 wt.% of levulinate ester. In some embodiments, the amount of levulinate ester in the composition may be less than 99.99 wt.%, less than 99.95 wt.% or less than 99.90 wt.%, based on the total weight of the composition.

According to the present invention, the levulinate ester is a levulinate ester with one ester group (also called levulinate monoester). The levulinate ester may also be a dilevulinate ester, which is a compound formed when levulinate ester is esterified with a glycol. It may also be a multilevulinate ester, which is a compound formed when levulinate ester is esterified with a polyol (alcohol with 2 or more hydroxyl groups), for example with sorbitol. Preferably, the levulinate ester is a levulinate monoester or a mixture of levulinate monoesters.

The composition of the present invention may comprise several distinct levulinate esters, for example two, three or four distinct levulinate esters. The composition of the present invention may comprise one levulinate ester only. The composition of the present invention may comprise a mixture of levulinate esters.

The composition of the present invention also comprises at least one phenolic antioxidant.

In some embodiments, the phenolic antioxidant constitutes the minor component in the composition. In other words, the phenolic antioxidant forms a small or lesser portion of the overall composition. It is not the primary or major component but is present in a smaller quantity relative to the levulinate ester component and other optional components in the mixture. For example, the amount of phenolic antioxidant may be from 10 ppm to 5,000 ppm, based on the total weight of the composition.

In some other embodiments, the amount of phenolic antioxidant in the composition is from 50 ppm to 3,000 ppm, from 100 ppm to 2,000 ppm or from 200 ppm to 1,500 ppm, based on the total weight of the composition.

The composition of the present invention may comprise several distinct phenolic antioxidants, for example two, three or four distinct phenolic antioxidants, for example a combination of distinct phenolic antioxidants.

The composition of the present invention may comprise several distinct antioxidants, wherein at least one of them is a phenolic antioxidant according to the present invention. For example, the composition of the present invention may comprise a combination of an antioxidant (or several) qualifying as a phenolic antioxidant and an antioxidant (or several) which does not qualify as such. Examples of anti-oxidant which are not phenolic antioxidant according to the present invention, but which could be combined to at least one phenolic antioxidants within the scope of the present invention, include, but are not limited to glutathione (GSH), ascorbic acid and its salts (vitamin C), ascorbyl esters of fatty acids, ascorbic acid derivatives, e.g., magnesium ascorbyl phosphate, sodium ascorbyl phosphate, ascorbyl sorbate, tetrahexyldecyl ascorbate, ascorbyl palmitate, ascorbyl tetrisopalmitate, 3-o-ethyl-L-ascorbic acid, ascorbyl glucoside) and mixtures thereof.

In the context of the present invention, more precisely with respect to combinations of antioxidants, the term "combination" herein involves blending several antioxidants to achieve a synergistic effect where the properties of each antioxidant enhance the effects of the others.

The composition of the present invention preferably comprises, or consists essentially of, one levulinate ester and one or several phenolic antioxidants.

In some embodiments, the composition of the present invention comprises vitamin E or derivative (at least) as the phenolic antioxidant.

In some embodiments, the phenolic antioxidant may be one of tocopherol, a derivative of tocopherol, tocotrienol, hydroquinone, an alkyl phenol, butylated hydroxy toluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and mixtures or combinations thereof. For sake of clarity, any derivative of these antioxidants, as defined in the context of the present invention, is meant to be encompassed within the scope of the present invention.

More specifically, the derivative of tocopherol may be an ester of tocopherol, such as tocopheryl sorbate, tocopheryl phosphate, tocopheryl acetate, tocopheryl nicotinate, tocopheryl linolate, tocopheryl palmitate, or mixtures thereof.

There are various forms of tocotrienol which are suitable as antioxidant in the present composition, more precisely α-tocotrienol (alpha-tocotrienol), β- tocotrienol (beta-tocotrienol), γ-tocotrienol (gamma-tocotrienol) and δ-tocotrienol (delta- tocotrienol).

The phenolic antioxidant is preferably at least one of tocopherol, a derivative of tocopherol, tocotrienol, hydroquinone, butylhydroxytoluene (BHT), and mixtures or combinations thereof. The phenolic antioxidant used in the compositions of the present invention is more preferably tocopherol. There are various forms of tocopherol, including α-tocopherol (alpha-tocopherol), β-tocopherol (beta-tocopherol), γ-tocopherol (gamma-tocopherol), δ-tocopherol (delta-tocopherol) and tocopherol acetate.

In some embodiments, the phenolic antioxidant is at least tocopherol (any form or derivative described above) which is used alone or in combination with another antioxidant or several other antioxidants (phenolic or not). Tocopherol products are commercially available.

The composition of the present invention may comprise other components and/or impurities.

The term "component" refers to a substance that is intentionally included in the composition. Other components may be present in the composition of the present invention; if they are included in the composition of the present invention, they are typically included in low amounts, for example less than 5 wt.% or less than 4 wt.% (and even lower).

In contrast, the term "impurity" refers to a substance that is not intentionally included in the composition and which is usually present in a very low amount. Impurities can arise from various sources, such as raw materials, manufacturing processes, or storage conditions. While impurities may affect the quality, safety, or efficacy of the final product, it is generally acceptable that compositions comprise low amounts of such impurities. The amount of such impurities and the nature of such impurities generally depend on the intended application, as different applications may tolerate varying levels of impurities based on the required quality standards and performance criteria.

In some embodiments, the composition of the present invention comprises less than 50,000 ppm of impurities, for example less than 20,000, less than 10,000 or less than 5,000, based on the total weight of the composition.

The composition of the present invention may comprise one or several impurities arising from the raw materials and manufacturing processes used for preparing the levulinate ester. The composition of the present invention generally comprises at least one such impurity.

Examples of impurities which may be present in the composition of the invention are water, lactone, levulinic acid, including derivatives of levulinic acid, levulinic ester, furanics, including functionalized pseudo-ethyl levulinate, and angelicalactone. This list is, however, not meant to be limiting.

In some embodiments, the levulinate ester is obtained from the esterification of levulinic acid with alcohols. Levulinic acid is typically derived from biomass, especially through the degradation of cellulose. This process involves treating cellulose with acid catalysts to produce levulinic acid, along with other by-products. Levulinic acid can be transformed into a wide range of valuable derivatives, including levulinate esters, gamma-valerolactone, methyltetrahydrofuran, diphenolic acid and 5-aminolevulinic acid.

As explained above, levulinate esters can be produced through several methods. The methods and raw materials used to produce the levulinate esters impact the purity of the final product, including the color of the final product, as well as the color stability of the product over time.

The esterification of levulinic acid with alcohols to prepare levulinate esters typically requires the presence of an acid catalyst (such as sulfuric acid) to accelerate the process and improve the yield of the ester. The reaction is usually carried out under reflux conditions to ensure complete conversion. The alcohol used in the esterification process is not limited. It may be either a monohydric alcohol or a polyhydric alcohol (alcohol having multiple hydroxyl groups). Monohydric alcohols, such as methanol, ethanol, propanol, and butanol, can for example be used. Polyhydric alcohols, including ethylene glycol, propylene glycol, and glycerol, can also be used to prepare other levulinate ester products, such as di- or triesters. Higher alcohols and branched alcohols, such as isopropanol, isobutanol, and tert-butanol, yield esters with branched chains. Within the scope of the present invention, the levulinate ester can also result from the esterification of levulinic acid with unsaturated alcohols, like allyl alcohol, and aromatic alcohols, such as phenol. Specialty alcohols, including cetyl and stearyl alcohols, can be used for application in cosmetics and personal care products ; sugar alcohols like sorbitol and mannitol can be used to prepare biocompatible and biodegradable esters.

In some embodiments, the levulinate ester used in the composition of the present invention derives from the esterification of levulinic acid with a monohydric or a polyhydric alcohol, for example having a number of hydroxyl groups ranging from 1 to 20, for example from 1 to 10 or from 1 to 5.

In some preferred embodiments, the alcohols used for preparing the levulinate ester is ethanol (ethyl levulinate), butanol (butyl levulinate), isoamyl alcohol (isoamyl levulinate), isobutanol (isobutyl levulinate), or 2-ethylhexanol (ethylhexyl levulinate).

Under certain conditions, the reaction may be represented according to the following equations: 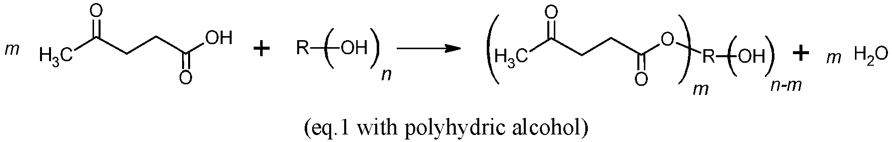 or 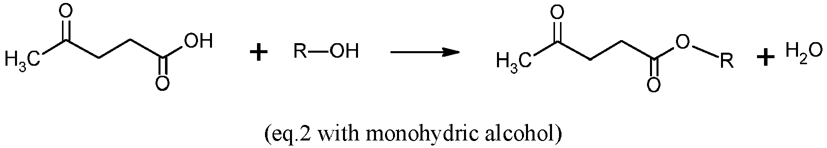 wherein:
R is substituted or unsubstituted aliphatic, aryl, aralkyl, alkaryl, heteroaralkyl, alkheteroaryl, or heteroaryl group, preferably a substituted or unsubstituted C1-C20 aliphatic group;
m is an integer ≥ 1, preferably m ranges from 1 to 20, from 1 to 10 or from 1 to 6;
n is an integer ≥ 1, preferably n ranges from 1 to 20, from 1 to 10 or from 1 to 6.

In some preferred embodiments, in equations 1-2, as well as equations 3-4 below, R is C₂H₅ (ethanol), C₄H₉ (butanol), C₅H₁₁ (isoamyl alcohol), C₄H₉ (branched, isobutanol), or C₈H₁₇ (2-ethylhexanol).

In some embodiments, the levulinate ester is obtained from the conversion of furfuryl alcohol in the presence of alcohols (e.g., methanol, ethanol, etc.). Alcohols which can be used according to these embodiments are as described above. Furfuryl alcohol is an organic compound derived from the hydrogenation of furfural. Furfural may typically be obtained from the processing of biomass, such as agricultural waste. Furfuryl alcohol contains a furan ring C₄H₃O with an attached hydroxymethyl group. The conversion or furfuryl alcohol typically takes place under acidic conditions, which act as a catalyst.

Under certain conditions, the reaction may be represented according to the following equations: 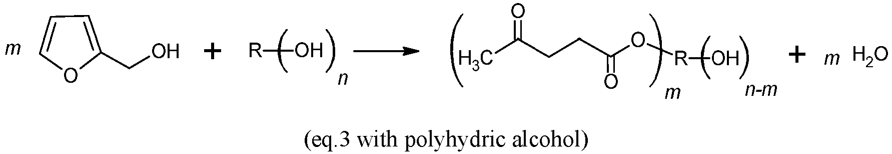 or 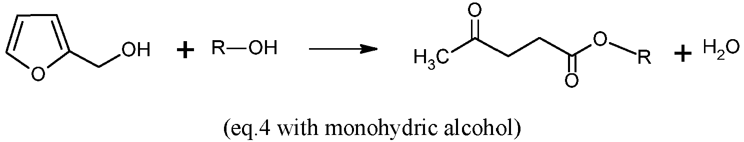 wherein:
R is substituted or unsubstituted aliphatic, aryl, aralkyl, alkaryl, heteroaralkyl, alkheteroaryl, or heteroaryl group, preferably a substituted or unsubstituted C1-C20 aliphatic group;
m is an integer ≥ 1, preferably m ranges from 1 to 20, from 1 to 10 or from 1 to 6;
n is an integer ≥ 1, preferably n ranges from 1 to 20, from 1 to 10 or from 1 to 6.

For example, the conversion of furfuryl alcohol in the presence of methanol produces methyl levulinate; the conversion of furfuryl alcohol in the presence of ethanol produces ethyl levulinate.

In some embodiments, the levulinate ester is obtained from the transesterification of levulinic acid-based esters. The reaction is generally conducted in the presence of an acid or base catalyst. This reaction involves exchanging the alkoxy group of a first ester with the alcohol group, forming a new ester and a by-product alcohol. Alcohols which can be used according to these embodiments are as described above. They can be monohydric alcohols or polyhydric alcohols.

Under certain conditions, the reaction may be represented according to the following equation: 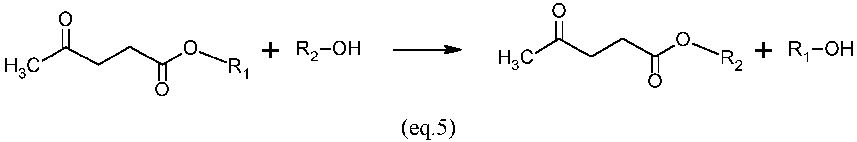 wherein:
R₁ and R₂ are distinct and independently substituted or unsubstituted aliphatic, aryl, aralkyl, alkaryl, heteroaralkyl, alkheteroaryl, or heteroaryl group, preferably a substituted or unsubstituted C1-C30 aliphatic group.

In some preferred embodiments, in equation 5, R₁ and R₂ are independently C₂H₅ (ethanol), C₄H₉ (butanol), C₅H₁₁ (isoamyl alcohol), C₄H₉ (branched, isobutanol), or C₈H₁₇ (2-ethylhexanol).

According to one example, methyl levulinate is converted into ethyl levulinate in the presence of ethanol.

In some embodiments, the levulinate ester is a byproduct of the synthesis of 5-methoxymethyl furfuraldehyde (5-MMF) or derivative thereof (e.g., 5-alkoxymethyl furfuraldehyde). More precisely, the conversion of sugars such as fructose to 5-MMF involves dehydration of fructose to form furfural typically achieved through the use of acid catalysts, and subsequently the methylation of furfural with methanol under acidic conditions to introduce a methoxymethyl group at the 5-position of the furan ring to lead to 5-MMF. In these embodiments, levulinate esters are the by-product arising from the reaction conditions used in the synthesis of 5-MMF.

The levulinate ester may preferably be an alkyl levulinate. In some embodiments, the levulinate ester is methyl levulinate, ethyl levulinate, butyl levulinate, isoamyl levulinate, isobutyl levulinate, ethylhexyl levulinate or a mixture thereof.

The inventors have found that adding a phenolic antioxidant to levulinate ester products has an effect in minimizing or preventing yellowing of these products. The solution has also been shown to attenuate the increase in yellow color over time (e.g., after 35 days).

In some embodiments, the composition of the present invention presents a color on Alpha Scale of less than 100, after 50 days of storage at room temperature, as measured using Lovibond^{®} EComparator 2000 for Pt-Co APHA Color scale, according to ASTM D1209. For example, the color is less than 90, less than 70, less than 50, or less than 40 after 50 days of storage at room temperature.

Levulinate ester compositions may be stored at high temperatures (i.e., temperatures higher than room temperature, e.g., temperatures above 30°C). Therefore, it is important that the technical effect of the phenolic antioxidant on the levulinate ester compositions remains true across different temperature ranges.

In some embodiments, the composition of the present invention presents a color on Alpha Scale of less than 180, after 35 days of storage at 50°C, as measured using Lovibond^{®} EComparator 2000 for Pt-Co APHA Color scale, according to ASTM D 1209. For example, the color is less than 170, less than 160, less than 165, or less than 160, after 35 days of storage at 50°C.

In some embodiments, the composition of the present invention comprises 0.05 to 5 mmol/L of phenolic antioxidant(s). For example, the composition may comprise from 0.1 to 4 mmol/L, from 0.15 to 3 mmol/L or from 0.2 to 2 mmol/L of phenolic antioxidant(s).

### Method for preparing the levulinate ester composition

The composition of the present invention can be prepared using a variety of mixing equipment, with the choice of equipment depending on the volume of the composition being produced. For example, laboratory-scale mixers may be used for small-scale preparations, while high-shear mixers are suitable for applications requiring thorough mixing and dispersion. For larger scale production, industrial blenders, such as ribbon blenders or fluidized bed mixers stirred vessels or static mixers, can be employed. Additionally, continuous mixers may be utilized for processes that involve constant production and blending of large volumes.

### Other aspects

The present invention also generally relates to the use of at least one phenolic antioxidant to reduce or avoid the color development in levulinate ester compositions.

All the embodiments described above in relation to the compositions apply similar to the present aspect of the invention.

The color-stable levulinate ester compositions described herein may be used in many and diverse end-use applications. The following markets can be mentioned, without the list being intended to be limited in scope: pesticides, herbicides, soil conditioners, drug delivery systems, pharmaceutical compositions, flavoring agents, food additives, cosmetics, personal care, fragrances, solvents, plasticizers, biofuels.

### ASPECTS OF THE INVENTION

The invention may be according to the following aspects:
Aspect 1. A levulinate ester composition comprising:
   - a levulinate ester; and
   - at least one phenolic antioxidant.
Aspect 2. The composition of aspect 1, wherein the amount of levulinate ester in the composition is at least 95 wt.%, based on the total weight of the composition, preferably at least 98 wt.%, 98.5 wt.%, at least 99 wt.%, at least 99.5 wt.%, based on the total weight of the composition.
Aspect 3. The composition of any one of aspects 1-2, wherein the amount of phenolic antioxidant is from 10 ppm to 5,000 ppm, based on the total weight of the composition, preferably from 50 ppm to 3,000 ppm, from 100 ppm to 2,000 ppm or from 200 ppm to 1,500 ppm, based on the total weight of the composition.
Aspect 4. The composition of any one of aspects 1-3, wherein the phenolic antioxidant comprises at least one of tocopherol, a derivative of tocopherol, tocotrienol, hydroquinone, an alkyl phenol, butylated hydroxy toluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and any derivative of these compounds, and mixtures or combinations thereof.
Aspect 5. The composition of aspect 4, wherein the derivative of tocopherol is an ester of tocopherol such as tocopheryl sorbate, tocopheryl acetate, tocopheryl phosphate, tocopheryl nicotinate, tocopheryl linolate, tocopheryl palmitate or mixtures thereof.
Aspect 6. The composition of aspect 4, wherein the phenolic antioxidant is at least tocopherol, which is used alone or in combination with another antioxidant.
Aspect 7. The composition of any one of aspects 1-6, wherein the levulinate ester is obtained from the esterification of levulinic acid with alcohols.
Aspect 8. The composition of any one of aspects 1-6, wherein the levulinate ester is obtained from the conversion of furfuryl alcohol in the presence of alcohols.
Aspect 9. The composition of any one of aspects 1-6, wherein the levulinate ester is obtained from the transesterification of levulinic acid-based esters.
Aspect 10. The composition of any one of aspects 1-6, wherein the levulinate ester is a byproduct of the synthesis of 5-alkoxymethyl furfuraldehyde-or derivative thereof.
Aspect 11. The composition of any one of aspects 1-10, wherein the levulinate ester is an alkyl levulinate.
Aspect 12. The composition of any one of aspects 1-11, wherein the levulinate ester is methyl levulinate, ethyl levulinate, butyl levulinate, isoamyl levulinate, isobutyl levulinate, ethylhexyl levulinate or a mixture thereof.
Aspect 13. The composition of any one of aspects 1-12, wherein the composition comprises at least one of ethyl levulinate, butyl levulinate, isoamyl levulinate, isobutyl levulinate, ethylhexyl levulinate or a mixture thereof, and at least one phenolic antioxidant which is tocopherol, tocotrienol or a derivative of tocopherol.
Aspect 14. The composition of any one of aspects 1-13, wherein the composition presents a color on Alpha Scale of less than 100, after 50 days of storage at room temperature, as measured using Lovibond^{®} EComparator 2000 for Pt-Co APHA Color scale, according to ASTM D1209.
Aspect 15. The composition of any one of aspects 1-14, wherein the composition presents a color on Alpha Scale of less than 180, after 35 days of storage at 50°C, as measured using Lovibond^{®} EComparator 2000 for Pt-Co APHA Color scale, according to ASTM D1209.
Aspect 16. Use of at least one phenolic antioxidant to reduce or avoid the color development in levulinate ester compositions.
Aspect 17. The use of aspect 16, wherein the phenolic antioxidant is chosen among tocopherol, a derivative of tocopherol, tocotrienol, hydroquinone, an alkyl phenol, butylated hydroxy toluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and any derivative of these compounds, and mixtures or combinations thereof.
Aspect 18. The use of aspect 17, wherein the derivative of tocopherol is an ester of tocopherol such as tocopheryl sorbate, tocopheryl acetate, tocopheryl phosphate, tocopheryl nicotinate, tocopheryl linolate, tocopheryl palmitate or mixtures thereof.
Aspect 19. The use of aspect 16 or 17, wherein the phenolic antioxidant is at least tocopherol, which is used alone or in combination with another antioxidant.
Aspect 20. The use of any one of aspects 16-19, wherein the amount of phenolic antioxidant in the composition is from 10 ppm to 5,000 ppm, based on the total weight of the composition, preferably from 50 ppm to 3,000 ppm, from 100 ppm to 2,000 ppm or from 200 ppm to 1,500 ppm, based on the total weight of the composition.

Although the foregoing invention has been described in some detail by way of illustration and example for purposes of clarity of understanding, one of skill in the art will appreciate that certain changes and modifications may be practiced within the scope of the appended claims. In addition, each reference provided herein is incorporated by reference in its entirety to the same extent as if each reference was individually incorporated by reference. Where a conflict exists between the instant application and a reference provided herein, the instant application shall dominate.

### EXAMPLES

The following examples illustrate the invention without limiting it.

### Raw materials

The following components were used in the examples (Table 1):

**Table 1: raw materials**

| **Abbreviations or Trade Name** | **Full Name and Supplying Information** |
|---|---|
| Ethyl Levulinate | Production batches, manufacturing process as described in WO 2022/034235 A1 |
| Tocopherol | DL-α-tocopherol (≥ 96%), from BASF India Limited |

### Measurement method for color evolution

To measure the color evolution in the tested samples, a colorimeter Lovibond^{®} EComparator 2000 was used to determine the platinum-cobalt (Pt-Co) APHA Color scale, following ASTM D1209.

Samples were introduced into the quartz measuring cell of the colorimeter.

The APHA scale, also known as the Hazen scale, is a visual assessment method based on comparing the color of the sample to standard platinum-cobalt solutions. On this scale, distilled water has a value of 0, while platinum-cobalt solution has a value of 500. Sample colors can range from transparent and colorless to yellow.

### Example 1

Example 1 was conducted on a production batch (batch #1) of ethyl levulinate.

Two 250 mL samples of ethyl levulinate were collected from this batch for testing and analysis.

The first sample was treated with tocopherol (theoretical concentration: 1,000 ppm). The second sample was left untreated to compare the effect of tocopherol treatment on color evolution with the color of the untreated production batch.

After 9 days, the two 250 mL samples were divided into two 125 mL samples each to measure color evolution under two distinct conditions. Samples 1.1 (without tocopherol) and 1.2 (with tocopherol) were kept at room temperature. Samples 1.3 (without tocopherol) and 1.4 (with tocopherol) were placed in an oven at 50°C to accelerate the aging process. The aging procedure at 50°C enables the assessment of the product's stability over time (aging test), as ethyl levulinate tends to turn yellow over time, with a more pronounced and accelerated change observed in the oven.

Day 0 in Table 2 below corresponds to the initial color measurement of all the samples before two of the samples were placed in the oven at 50°C for the ageing test (Day 0 = 9 days after tocopherol addition to prepare samples 1.2 and 1.4). The difference in color between tocopherol-treated and untreated samples (53 vs. 39) is attributed to the fact that tocopherol has already begun to affect the color over the first 9-day time period.

**Table 2: Color evolution over time of treated and untreated ethyl levulinate samples from batch #1 with tocopherol, at room temperature or at 50°C.**

| Samples | Treatment | Storage condition | Color on APHA scale | | | | | |
|---|---|---|---|---|---|---|---|---|
| | | | Day 0 | Day 7 | Day 14 | Day 21 | Day 28 | Day 35 |
| 1.1 (comp) | without tocopherol | room temperature | 53 | 63 | 79 | 90 | 110 | 115 |
| 1.2 (inv) | with tocopherol | room temperature | 39 | 36 | 44 | 41 | 35 | 34 |
| 1.3 (comp) | without tocopherol | 50°C | 53 | 175 | 250 | 300 | 350 | 375 |
| 1.4 (inv) | with tocopherol | 50°C | 39 | 120 | 120 | 105 | 110 | 113 |

| | | | | | | | | |
|---|---|---|---|---|---|---|---|---|
| "inv" means according to the invention; "comp" means comparative. | | | | | | | | |

### Results:

At room temperature, the color of inventive sample 1.2 (treated with tocopherol) remained stable over time, while the color of the comparative untreated sample 1.1 increased almost fourfold from 53 to 185 after two months.

At 50°C, the color of both untreated and tocopherol-treated samples increased. However, while color increased by a factor of about 7 for the untreated sample 1.3 (i.e., from 53 to 375) at Day 35, it only increased by a factor of less than 3 for the treated sample 1.4 (i.e., from 39 to 113) on the same day.

This set of tests demonstrate that tocopherol not only presents the effect of preventing yellowing of ethyl levulinate samples at room temperature, but also the effect of significantly attenuating the increase in color at 50°C compared to untreated samples.

### Example 2

Example 1 was repeated on two additional production batches (batch #2 and batch #3) of ethyl levulinate.

Batch #2 was a 200-L drum of ethyl levulinate and batch #3 was a 950-L IBC of ethyl levulinate. Samples were collected from each of these batches for testing and analysis. The first sample from each batch was treated with tocopherol (theoretical concentration: 1,000 ppm). The second sample was left untreated to compare the effect of tocopherol treatment on color evolution with the color of the untreated production batch. Treated samples from batch #2 showed a tocopherol concentration of 894 ppm, i.e., 2.07 mmol/L. Treated samples from batch #3 showed a tocopherol concentration of 912 ppm, i.e., 2.12 mmol/L. These concentrations were determined by external calibration using GC-FID. More precisely, the quantification was performed using a Perkin Elmer GC 2400 system, equipped with an Elite-5 column (30 m x 0.25 mm x 0.25 µm). The calibration curve was established with an α-tocopherol analytical standard from Sigma Aldrich (purity > 95.5%) at concentrations ranging from 0 to 2000 ppm. The GC temperature program started at 60°C during 3.5 min, followed by a ramp of 20°C/min up to reach 300°C, which was held for 10 min. The carrier gas was helium at a flow rate of 1 mL/min. The injection volume was 1 µL and the split flow 50 mL/min, in an injector set at 250°C. The temperature detector was 310°C, with a fuel flow (H₂) of 30 mL/min, oxidizer flow (air) of 400 mL/min and makeup flow (N2) of 25 mL/min.

### Test on samples from batch #2

After 8 days, the 200 mL samples from batch #2 were divided into two 100 mL samples, one of being kept at room temperature and the other one being placed in an oven at 50°C. Results of the comparative color measurements are shown in Table 3 below.

Day 0 in Table 3 below corresponds to the initial color measurement of all the samples before two of the samples were placed in the oven at 50°C for the ageing test (Day 0 = 8 days after tocopherol addition to prepare samples 2.2 and 2.4). The difference in color between tocopherol-treated and untreated samples is attributed to the fact that tocopherol has already begun to affect the color over the first 8 days.

**Table 3: Color evolution over time of treated and untreated ethyl levulinate samples from batch #2 with tocopherol, at room temperature or at 50°C.**

| Samples | Treatment | Storage condition | Color on APHA scale | | | | | | |
|---|---|---|---|---|---|---|---|---|---|
| | | | Day 0 | Day 4 | Day 5 | Day 6 | Day 11 | Day 14 | Day 27 |
| 21 (comp) | without tocopherol | room temperature | 41 | 42 | 45 | 45 | 58 | 65 | 79 |
| 22 (inv) | with tocopherol | room temperature | 22 | 18 | 20 | 20 | 23 | 24 | 28 |
| 23 (comp) | without tocopherol | 50°C | 41 | 91 | 100 | 105 | 130 | 155 | 210 |
| 2.4 (inv) | with tocopherol | 50°C | 22 | 53 | 58 | 48 | 44 | 44 | 50 |

### Test on samples from batch #3

After 6 days, the 200 mL samples from batch #3 were divided into two 100 mL samples, one of being kept at room temperature and the other one being placed in an oven at 50°C. Results of the comparative color measurements are shown in Table 4 below.

Day 0 in Table 4 below corresponds to the initial color measurement of all the samples before two of the samples were placed in the oven at 50°C for the ageing test (Day 0 = 6 days after tocopherol addition to prepare samples 3.2 and 3.4). The difference in color between tocopherol-treated and untreated samples is attributed to the fact that tocopherol has already begun to affect the color over the first 8 days.

**Table 4: Color evolution over time of treated and untreated ethyl levulinate samples from batch #3 with tocopherol, at room temperature or at 50°C.**

| Samples | Treatment | Storage condition | Color on APHA scale | | | | | | |
|---|---|---|---|---|---|---|---|---|---|
| | | | Day 0 | Day 4 | Day 5 | Day 6 | Day 11 | Day 14 | Day 27 |
| 2.5 (comp) | without tocopherol | room temperature | 18 | 28 | 33 | 30 | 43 | 52 | 74 |
| 26 (inv) | with tocopherol | room temperature | 16 | 19 | 17 | 19 | 23 | 26 | 27 |
| 27 (comp) | without tocopherol | 50°C | 18 | 81 | 91 | 85 | 110 | 140 | 215 |
| 28 (inv) | with tocopherol | 50°C | 16 | 58 | 66 | 70 | 75 | 68 | 70 |

The results in Tables 3 and 4 using two distinct batches of levulinate esters demonstrate similar trends to those observed in Example 1 under both conditions: tocopherol has an effect in preventing yellowing of ethyl levulinate samples at room temperature, but also in significantly attenuating the increase in color at 50°C compared to untreated samples.

### Example 3

### Example 3 was conducted on a production batch (batch #4) of ethyl levulinate.

To carry out a reproducibility test, samples of batch #4 were prepared by two different operators.

Approximately 100 mL of ethyl levulinate from batch #4 was collected for each of the 8 samples. 4 of these samples remained untreated, while the 4 other ones were treated with about 1,000 ppm of tocopherol. As for the tests conducted in Examples 1 and 2, part of the samples was placed in the oven and the others were kept at room temperature. Table 5 below shows the concentration of tocopherol in ppm and mmol/L for the treated samples.

Day 0 in Table 5 below corresponds to the initial color measurement of all the samples before two of the samples were placed in the oven at 50°C for the ageing test. Unlike the other tests conducted in Examples 1 and 2, the ageing test was initiated on the same day as the tocopherol treatment. The difference in color between samples at Day 0 is attributable to analytical error and represents less than 6%.

**Table 5: Color evolution over time of treated and untreated ethyl levulinate samples from batch #4 with tocopherol, at room temperature or at 50°C.**

| Sample | Treatment | Storage condition | Tocopherol Concentration | | Color on APHA scale | | | | | | | | | |
|---|---|---|---|---|---|---|---|---|---|---|---|---|---|---|
| | | | (ppm) | (mmol/L) | D0 | D1 | D3 | D5 | D6 | D7 | D9 | D13 | D14 | D15 |
| 3.1 | without tocopherol | room temp | | | 59 | 79 | 91 | 105 | 115 | 115 | 120 | 125 | 130 | 125 |
| 3.2 | with tocopherol | room temp | 1018 | 2.38 | 62 | 85 | 100 | 90 | 80 | 80 | 89 | 81 | 79 | 72 |
| 3.3 | without tocopherol | 50°C | | | 59 | 93 | 120 | 135 | 180 | 185 | 235 | 260 | 270 | 260 |
| 3.4 | with tocopherol | 50°C | 1018 | 238 | 62 | 85 | 73 | 69 | 55 | 53 | 57 | 55 | 54 | 45 |
| | | | | | D0 | D1 | D3 | D5 | D6 | D7 | D9 | D13 | D14 | |
| 3.1 bis | without tocopherol | room temp | | | 59 | 95 | 105 | 100 | 105 | 105 | 105 | 110 | 98 | |
| 3.2 bis | with tocopherol | room temp | 998 | 2.34 | 62 | 70 | 95 | 90 | 85 | 83 | 77 | 76 | 71 | |
| 3.3 bis | without tocopherol | 50°C | | | 61 | 95 | 96 | 110 | 120 | 150 | 180 | 195 | 190 | |
| 3.4 bis | with tocopherol | 50°C | 1002 | 235 | 62 | 70 | 87 | 70 | 65 | 59 | 52 | 54 | 34 | |

Samples 3.1 and 3.1 bis (without tocopherol at room temperature) exhibited a significant increase in color over 15 days. Similarly, samples 3.3 and 3.3 bis (without tocopherol at 50°C) also showed an increase, with an even higher color value at Day 15.

The effect of tocopherol is very noticeable, stabilizing color as shown by the reproducibility test. After two weeks, the color of the two samples treated with tocopherol A at room temperature increased slightly from 62 to only 71-72. At 50°C, the color of the two tocopherol-treated samples, which started out at 62, even decreased after two weeks, reaching 45 and 34.

## Claims

1. A levulinate ester composition comprising:
- a levulinate ester; and
- at least one phenolic antioxidant.

2. The composition of claim 1, wherein the amount of levulinate ester in the composition is at least 95 wt.%, based on the total weight of the composition.

3. The composition of any one of claims 1-2, wherein the amount of phenolic antioxidant is from 10 ppm to 5,000 ppm, based on the total weight of the composition.

4. The composition of any one of claims 1-3, wherein the phenolic antioxidant comprises at least one of tocopherol, a derivative of tocopherol, tocotrienol, hydroquinone, an alkyl phenol, butylated hydroxytoluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and any derivative of these compounds, and mixtures or combinations thereof.

5. The composition of claim 4, wherein the derivative of tocopherol is an ester of tocopherol such as tocopheryl sorbate, tocopheryl acetate, tocopheryl phosphate, tocopheryl nicotinate, tocopheryl linolate, tocopheryl palmitate or mixtures thereof.

6. The composition of any one of claims 1-5, wherein the levulinate ester is obtained from the esterification of levulinic acid with alcohols.

7. The composition of any one of claims 1-5, wherein the levulinate ester is obtained from the conversion of furfuryl alcohol in the presence of alcohols.

8. The composition of any one of claims 1-5, wherein the levulinate ester is obtained from the transesterification of levulinic acid-based esters.

9. The composition of any one of claims 1-5, wherein the levulinate ester is a byproduct of the synthesis of 5-alkoxymethyl furfuraldehyde-or derivative thereof.

10. The composition of any one of claims 1-9, wherein the levulinate ester is an alkyl levulinate.

11. The composition of any one of claims 1-10, wherein the levulinate ester is methyl levulinate, ethyl levulinate, butyl levulinate, isoamyl levulinate, isobutyl levulinate, ethylhexyl levulinate or a mixture thereof.

12. The composition of any one of claims 1-11, wherein the composition presents a color on Alpha Scale of less than 100, after 50 days of storage at room temperature, as measured using Lovibond^{®} EComparator 2000 for Pt-Co APHA Color scale, according to ASTM D1209.

13. The composition of any one of claims 1-12, wherein the composition presents a color on Alpha Scale of less than 180, after 35 days of storage at 50°C, as measured using Lovibond^{®} EComparator 2000 for Pt-Co APHA Color scale, according to ASTM D1209.

14. Use of at least one phenolic antioxidant to reduce or avoid the color development in levulinate ester compositions.

15. Use of claim 14, wherein the phenolic antioxidant comprises at least one of tocopherol, a derivative of tocopherol, tocotrienol, hydroquinone, an alkyl phenol, butylated hydroxy toluene (BHT), butylated hydroxyanisole (BHA), tertiary butylated hydroxyquinone (TBHQ), propyl gallate (PG), octyl gallate (OG), dodecyl gallate (DG), a catechin, benzoic acid, hydroxybenzoic acid, dihydroxybenzoic acid, p-hydroxybenzoic acid and derivatives (Bas, p-HBAs, DHBAs ), vanillic acid (VA), gallic acid (GA), salicylic acid, syringic acid, hydroxycinnamic acid and derivatives (e.g., caffeic acid, ferulic acid, sinapic acid, p-coumaric acid, o-coumaric acid), stilbenoid and derivatives of the aglycone type (e.g., resveratrol) and derivatives of the glycoside type (e.g., piceid), tannins and hydrolysable derivatives (e.g., ellagitannins, gallotannins) and condensates derivatives (e.g., proanthocyanidins), lignans, lignins, and any derivative of these compounds, and mixtures or combinations thereof.
